# EUROPEAN PATENT APPLICATION

(11) **EP 1 374 889 A1**
(43) Date of publication of application: **02.01.2004**
(21) Application number: 03016266.3
(22) Date of filing: 22.06.2000
(51) Int. Cl.: A61K 38/18, C12N 5/06

(54) **Method of inducing angiogenesis by micro-organs**

(30) Priority: 25.06.1999 US 140748
(62) Divisional of application: 00939024.6
(71) Applicant: YISSUM RESEARCH DEVELOPMENT COMPANY OF THE HEBREW UNIVERSITY OF JERUSALEM, 91042 Jerusalem (IL)
(72) Inventor: Mitrani, Eduardo N., 93510 Jerusalem (IL)
(74) Representative: Schmitz, Jean-Marie

(57) **Abstract**

A genetically modified micro-organ explant expressing at least one recombinant gene product, the micro-organ explant comprising a population of cells, the micro-organ explant maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the micro-organ explant, at least some of the cells of said population of cells of the micro-organ explant expressing at least one recombinant gene product.

SEQUENCE LISTING

<110> Eduardo N. Mitrani

<120> METHOD OF INDUCING ANGIOGENESIS BY micro-organs

<130> 20095

<150> 60/140,748

<151> June 25, 1999

<160> 8

<170> PatentIn Ver. 2.1

<210> 1

<211> 21

<212> DNA

<213> Artificial sequence

<220>

<223> Description of Artificial sequence: PCR primer

<400> 1

CGTGGGTGGA GGAGGGTGGA C 21

<210> 2

<211> 21

<212> DNA

<213> Artificial sequence

<220>

<223> Description of Artificial sequence: PCR primer

<400> 2

TGCGTCAAAC CACCAGCCTC C 21

<210> 3

<211> 20

<212> DNA

<213> Artificial sequence

<220>

<223> Description of Artificial sequence: PCR primer

<400> 3

TACCACAGGC ATTGTGATGG 20

<210> 4

<211> 18

<212> DNA

<213> Artificial sequence

<220>

<223> Description of Artificial sequence: PCR primer

<400> 4

AATAGTGATGA CCTGGCCGT 18

<210> 5

<211> 20

<212> DNA

<213> Artificial sequence

<220>

<223> Description of Artificial sequence: PCR primer

<400> 5

GGTCACACAG GGACAGCAGG 20

<210> 6

<211> 21

<212> DNA

<213> Artificial sequence

<220>

<223> Description of Artificial sequence: PCR primer

<400> 6

CCAAGGGCCG GATCAGCATG G 21

<210> 7

<211> 19

<212> DNA

<213> Artificial sequence

<220>

<223> Description of Artificial sequence: PCR primer

<400> 7

ACTTTCTGCT CTCTTGGGT 19

<210> 8

<211> 18

<212> DNA

<213> Artificial sequence

<220>

<223> Descriptionof Artificial sequence: PCR primer

<400> 8

CCGCCTTGGC TTGTCACA 18

## Description

### FIELD AND BACKGROUND OF THE INVENTION

The present invention relates to methods of utilizing micro-organs, extracts derived therefrom and pharmaceutical composition including said extracts for stimulating angiogenesis in host tissues. The present invention further relates to micro-organs transformed with exogenous nucleotide sequences and methods of utilizing same for providing angiogenic factors to host tissues.

During the last few years numerous research studies have provided new insights into the molecular mechanisms which induce and regulate angiogenesis. The discovery of angiogenic growth factors such as vascular endothelial growth factor (VEGF), basic fibroblast growth factor (bFGF), angiopoietin and others, has led researchers to consider the use of these factors as agents for revascularize ischemic tissue regions. Several different approaches utilizing either gene therapy or recombinant protein technology have been attempted. Although preliminary results in animals were promising, clinical tests so far conducted, have produced disappointing results (Ferrara and Alitalo, 1999 *Nature Medicine* 5(12): 1359-1364).

The lack of success at the clinical level, can be attributed, at least in part, to the gene therapy or recombinant protein technology utilized in these experiments.

It has been shown that *in-vivo* angiogenesis is effected and regulated by a complex and dynamic set of factors, including both stimulators and inhibitors (see Iruela-Arispe and Dvorak, 1997 *Thrombosis and Haemostasis* 78(1), 672-677, Gale and Yancopolous, 1999 *Genes and Development* 13, 1055-1066). In addition, it is thought that a long term sustained stimulus is required to induce angiogenesis. Therefore, the current gene therapy and recombinant growth factors techniques which do not address these issues cannot produce the conditions necessary for promoting *in-vivo* angiogenesis.

Recently, the inventors of the present invention have described a method for producing micro organs which can be sustained outside the body in an autonomously functional state for extended periods of time. Such micro-organs, their preparation, preservation and some uses thereof are described, for example, in U.S. Pat. No. 5,888,720; U.S. Pat. Application No. 09/425,233, and in PCT/US98/00594, which are incorporated herein by reference, and in the preferred embodiments section hereinbelow.

While reducing the present invention to practice, and as is further detailed in the Examples section below, it was uncovered that when such micro-organs are implanted within living tissue, they provide the tissue with a sustained and complex repertoire of regulated angiogenic factors which lead to a marked induction of new blood vessels. It was also discovered that implanted micro-organs can resurrect surgically-induced ischemic areas in both normal and aging animals.

Thus, the present invention provides a novel and effective method of inducing angiogenesis, which method can be utilized for treating ischemic tissues thus traversing the limitations of prior art methods.

### SUMMARY OF THE INVENTION

According to one aspect of the present invention there is provided a method of inducing angiogenesis in a tissue of a first mammal, the method comprising the step of implanting at least one micro-organ within the tissue of the first mammal, the at least one micro-organ being for producing a plurality of angiogenic factors and thereby inducing angiogenesis.

According to another aspect of the present invention there is provided a method of inducing angiogenesis in a tissue of a first mammal, the method comprising the steps of (a) extracting soluble molecules from at least one micro-organ; and (b) administering at least one predetermined dose of the soluble molecules extracted in step (a) into the tissue of the first mammal.

According to yet another aspect of the present invention there is provided a micro-organ comprising a plurality of cells, wherein at least a portion of the plurality of the cells including at least one exogenous polynucleotide sequence, the at least one exogenous polynucleotide sequence being capable of regulating expression of at least one angiogenic factor expressed in the cells.

According to still another aspect of the present invention there is provided a pharmaceutical composition comprising a soluble molecule extract from at least one micro-organ and a pharmaceutically acceptable carrier.

According to still another aspect of the present invention there is provided a method of inducing angiogenesis in a tissue of a first mammal, the method comprising the steps of: (a) culturing at least one micro-organ in a growth medium to thereby generate a conditioned medium; (b) collecting the conditioned medium following at least one predetermined time period of culturing; and (b) administering at least one predetermined dose of the conditioned medium collected in step (b) into the tissue of the first mammal to thereby induce angiogenesis in the tissue.

According to further features in preferred embodiments of the invention described below, the growth medium is a minimal essential medium.

According to still further features in the described preferred embodiments the at least one micro-organ is derived from organ tissue of a second mammal.

According to still further features in the described preferred embodiments the first mammal and the second mammal are a single individual mammal.

According to still further features in the described preferred embodiments the organ is selected from the group consisting of a lung, a liver, a kidney, a muscle, a spleen a skin or any other internal organ.

According to still further features in the described preferred embodiments the at least one micro-organ includes two or more cell types.

According to still further features in the described preferred embodiments the first mammal is a human being.

According to still further features in the described preferred embodiments the at least one micro-organ is cultured outside the body for at least four hours prior to implantation within the tissue of the first mammal.

According to still further features in the described preferred embodiments the at least one micro-organ is prepared so as to retain viability when implanted within the tissue of the mammal.

According to still further features in the described preferred embodiments the at least one micro-organ has dimensions, such that cells positioned deepest within the at least one micro-organ are at least about 100 micrometers and not more than about 225-350 micrometers away from a nearest surface of the at least one micro-organ.

According to still further features in the described preferred embodiments each of the plurality of angiogenic factors posses a unique expression pattern within the at least one micro-organ.

According to still further features in the described preferred embodiments at least a portion of cells of the at least one micro-organ include at least one exogenous polynucleotide sequence selected for regulating angiogenesis.

According to still further features in the described preferred embodiments the at least one exogenous polynucleotide sequence is integrated into a genome of the at least a portion of the cells of the at least one micro-organ.

According to still further features in the described preferred embodiments the at least one exogenous polynucleotide sequence is designed for regulating expression of at least one angiogenic factor of the plurality of angiogenic factors.

According to still further features in the described preferred embodiments the at least one exogenous polynucleotide sequence includes an enhancer or a suppresser sequence.

According to still further features in the described preferred embodiments an expression product of the at least one exogenous polynucleotide sequence is capable of regulating the expression of at least one angiogenic factor of the plurality of angiogenic factors.

According to still further features in the described preferred embodiments the at least one exogenous polynucleotide sequence encodes at least one recombinant angiogenic factor.

The present invention successfully addresses the shortcomings of the presently known configurations by providing a method, extract and pharmaceutical composition useful for inducing angiogenesis in a tissue of a mammal.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a photograph showing neo-vascularization around an implanted micro-organ (marked with arrow).
FIG. 2 is a graph illustrating the relative levels of various angiogenic factors expressed in transplanted micro-organs. Ang1 - angiopoietin 1, Ang2 - angiopoietin 2, MEF2C - myocyte enhancer factor 2C, VEGF - vascular endothelial growth factor.
FIG. 3 is an angiogenic factor-specific RT-PCR of RNA extracted from micro-organs cultured outside the body for various time points following prepration. Actin - beta-actin (control).
FIG. 4 is a graph representing semi-quantitative data obtained by densitometry of the RT-PCR products shown in Figure 3, normalized to the intensity of the beta-actin RT-PCR product (control).
FIG. 5 is a histogram representing the gating pattern of common iliac-ligated rats implanted with micro-organs or sham implanted (control). (n) = 13. P values for the three time groups (from left to right) are 0.16, 1 and 0.841. Scores: 0-full functionality 9-total inability to move the limb, 10 loss of the limb.;
FIG. 6 is a histogram representing the same experimental group as in Figure 5 with the exception that the animals were now exerted prior to scoring gating behavior. P values for the three time groups are (from left to right) 0.0001, 0.0069 and 0.06.
FIG. 7 is a histogram representing the gating pattern of common iliac-ligated mice implanted with micro-organs or sham implanted. Scores: 0-full functionality 9-total inability to move the limb, 10 loss of the limb. P values for the three time groups are (from left to right) 0.00025, 0.00571 and 0.07362.
FIG. 8 is an image illustrating a mouse spleen derived micro-organ (marked with MC arrow) six months following implantation into a subcutaneous region of a syngeneic mouse. One of the newly formed blood vessels surrounding the micro-organ is marked with an arrow.
FIG. 9 is an image illustrating a rat cornea implanted with lung micro-organs from a syngeneic rat. The implanted micro-organ (marked with arrow) is surrounded by newly formed blood vessels.

### DESCRIPTION OF THE PREFERRED EMBODIMENTS

The present invention is of a method of utilizing micro-organs, extracts derived therefrom or pharmaceutical compositions including said extracts for inducing angiogenesis in mammalian tissues. Specifically, the present invention can be used to induce angiogenesis in mammalian tissue via either implantation of one or more micro-organs, or via local administration of a soluble extract derived therefrom which is preferably included within a pharmaceutical composition.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or exemplified in the examples section that follows. The invention is capable of other embodiments or of being practiced or carried out in various ways. Also, it is to be understood that the phraseology and terminology employed herein is for the purpose of description and should not be regarded as limiting.

As used herein, the term "micro-organ" refers to organ tissue which is removed from a body and which is prepared, as is further described below, in a manner conducive for cell viability and function. Such preparation may include culturing outside the body for a predetermined time period.

Complex multicellular organisms rely on a vascular network to support the needs of each and every cell for oxygen, nutrients and waste removal. This complex network of blood vessels is created and sustained through the process of angiogenesis. In humans, the deterioration of the vascular network leads to occlusive arterial disease, which is the leading cause for morbidity and mortality in the Western world. Most currently available therapeutic options are based on surgical or other invasive procedures, such as vascular bypass or angioplasty. These solutions are for the most part successful but may be short lived or not applicable to all patients. Since angiogenesis is a fundamental component of tissue and organ genesis, most tissues retain the capacity to induce new vessel formation during regeneration. Thus, the inventors of the present invention postulate that tissue which is removed from the body is in essence at least attempting to undergo regeneration and thus can be utilized as an angiogenic stimulant.

The present invention provides a new approach to induce angiogenesis, which approach is based on the use of micro organs. Such micro-organs retain the basic micro-architecture of the tissues of origin while at the same time are prepared such that cells of an organ explant are not more than 100-450 microns away from a source of nutrients and gases. Such micro-organs function autonomously and remain viable for extended period of time both as ex-vivo cultures and in the implanted state.

It will be appreciated that although micro-organs can be utilized immediately following preparation, in some cases culturing outside the body for extended periods of time may be advantageous in order to increase viability. For example, in cases where soluble molecules are to be extracted, culturing of micro-organ is performed for predetermined time periods, which can be as short as 4 hours or as long as days or weeks.

Thus, the use of these micro-organs or extracts derived therefrom for inducing angiogenesis is dependent on the preservation of the cellular function for various periods of time prior to implantation. The present invention is based, in part, upon the discovery that under defined circumstances growth of cells in different tissue layers of an organ explant, e.g., mesenchymal and epithelial layers, can be activated to proliferate, differentiate and function in culture.

The cell-cell and cell-matrix interactions provided in the explant itself are sufficient to support cellular homeostasis, thereby sustaining the microarchitecture and function of the organ for prolonged periods of time. As used herein, the term "homeostasis" is defined as an equilibrium between cell proliferation and cell loss.

The support of cellular homeostasis preserves, for example, the natural cell-cell and cell-matrix interactions occurring in the source organ. Thus, orientation of the cells with respect to each other or to another anchorage substrate, as well as the presence or absence of regulatory substances such as hormones, permits the appropriate maintenance of biochemical and biological activity of the source organ. Moreover, the micro-organ can be maintained in culture without significant necrosis for at least 48 days or longer.

### Source of explants for the micro-organ

Examples of mammals from which the micro-organs can be isolated include humans and other primates, swine, such as wholly or partially inbred swine (e.g., miniature swine, and transgenic swine), rodents, etc. Examples of suitable organs include, but are not limited to, liver, lung, other gut derived organs, heart, spleen, kidney, skin and pancreas.

### The growth media

There are a large number of tissue culture media that exist for culturing cells from animals. Some of these are complex and some are simple. While it is expected that micro-organs may grow in complex media, it has been shown in United States Patent Application Serial Number 08/482,364 that cultures can be maintained in a simple medium such as Dulbecco's Minimal Essential Media (DMEM). Furthermore, although the micro-organs may be cultured in a media containing sera or other biological extracts such as pituitary extract, it has been shown in United States Patent Application Serial Number 08/482,364 that neither sera nor any other biological extract is required. Moreover, the micro-organ cultures can be maintained in the absence of sera for extended periods of time. In preferred embodiments of the invention, growth factors are not included in the media during maintenance of the micro-organ cultures *in vitro.*

The point regarding growth in minimal media is important. At the present, most media or systems for prolonged growth of mammalian cells incorporate undefined proteins or use feeder cells to provide proteins necessary to sustain such growth. Because the presence of such undefined proteins can interfere with the intended end use of the micro-organs, it will generally be desirable to culture the explants under conditions to minimize the presence of undefined proteins.

As used herein the language "minimal medium" refers to a chemically defined medium which includes only the nutrients that are required by the cells to survive and proliferate in culture. Typically, minimal medium is free of biological extracts, e.g., growth factors, serum, pituitary extract, or other substances which are not necessary to support the survival and proliferation of a cell population in culture. For example, minimal medium generally includes at least one amino acid, at least one vitamin, at least one salt, at least one antibiotic, at least one indicator, e.g., phenol red, used to determine hydrogen ion concentration, glucose, and at least one antibiotic, and other miscellaneous components necessary for the survival and proliferation of the cells. Minimal medium is serum-free. A variety of minimal media are commercially available from Gibco BRL, Gaithersburg, MD, as minimal essential media.

However, while growth factors and regulatory factors need not be added to the media, the addition of such factors, or the inoculation of other specialized cells may be used to enhance, alter or modulate proliferation and cell maturation in the cultures. The growth and activity of cells in culture can be affected by a variety of growth factors such as insulin, growth hormone, somatomedins, colony stimulating factors, erythropoietin, epidermal growth factor, hepatic erythropoietic factor (hepatopoietin), and other cell growth factors such as prostaglandins, interleukins, and naturally-occurring negative growth factors, fibroblast growth factors, and members of the transforming growth factor-beta family.

### Culture Vessel

The micro-organs may be maintained in any suitable culture vessel and may be maintained at 37 °C in 5 % CO₂. The cultures may be shaken for improved aeration.

With respect to the culture vessel in/on which the micro-organs are preferably provided, it is noted that in a preferred embodiment such a vessel may generally be of any material and/or shape. A number of different materials may be used to form the vessel, including but not limited to: nylon (polyamides), dacron (polyesters), polystyrene, polypropylene, polyacrylates, polyvinyl compounds (e.g., polyvinylchloride), polycarbonate (PVC), polytetrafluorethylene (PTFE; teflon), thermanox (TPX), nitrocellulose, cotton, polyglycolic acid (PGA), cat gut sutures, cellulose, gelatin, dextran, etc. Any of these materials may be woven into a mesh.

Where the cultures are to be maintained for long periods of time or cryopreserved, non-degradable materials such as nylon, dacron, polystyrene, polycarbonate, polyacrylates, polyvinyls, teflons, cotton or the like may be preferred. A convenient nylon mesh which could be used in accordance with the invention is Nitex, a nylon filtration mesh having an average pore size of 210 µm and an average nylon fiber diameter of 90 µm (Tetko, Inc., N. Y.).

### Dimensions of the Explant

In addition to isolating an explant which retains the cell-cell, cell-matrix and cell-stroma architecture of the originating tissue, the dimensions of the explant are crucial to the viability of the cells therein, e.g., where the micro-organ is intended to be sustained for prolonged periods of time, such as 7-21 days or longer.

Accordingly, the dimensions of the tissue explant are selected to provide diffusion of adequate nutrients and gases such as oxygen to every cell in the three dimensional micro-organ, as well as diffusion of cellular waste out of the explant so as to minimize cellular toxicity and concomitant death due to localization of the waste in the micro-organ. Accordingly, the size of the explant is determined by the requirement for a minimum level of accessibility to each cell in the absence of specialized delivery structures or synthetic substrates. It has been discovered, as described in United States Patent Application Number 08/482,364, that this accessibility can be maintained if the surface to volume index falls within a certain range.

This selected range of surface area to volume index provides the cells access to nutrients and to avenues of waste disposal by diffusion in a manner similar to cells in a monolayer. This level of accessibility can be attained and maintained if the surface area to volume index, defined herein as "Aleph or Aleph index" is at least about 2.6 mm⁻¹. The third dimension has been ignored in determining the surface area to volume index because variation in the third dimension causes ratiometric variation in both volume and surface area. However, when determining Aleph, a and x should be defined as the two smallest dimensions of the tissue slice.

As used herein, "Aleph" refers to a surface area to volume index given by a formula 1/x+1/a, wherein x= tissue thickness and a= width of tissue in mm. In preferred embodiments, the Aleph of an explant is in the range of from about 2.7 mm⁻¹ to about 25 mm⁻¹, more preferably in the range of from about 2.7 mm⁻¹ to about 15 mm⁻¹, and even more preferably in the range of from about 2.7 mm⁻¹ to about 10 mm⁻¹.

Examples of Aleph are provided in Table 1 wherein, for example, a tissue having a thickness (x) of 0.1 mm and a width (a) of 1 mm would have an Aleph index of 11 mm⁻¹.

**TABLE 1**

| ***Different values for the surface area to volume ratio index "Aleph", as a function of a (width) and x (thickness) in mm***^{***-1***} | | | | | |
|---|---|---|---|---|---|
| x (mm) | Values of Aleph | | | | |
| | a=1 | a=2 | a=3 | a=4 | a=5 |
| 0.1 | 11 | 10.5 1 | 10.33 | 10.2 | 10.2 |
| 0.2 | 6 | 5.5 | 5.33 | 5.25 | 5.2 |
| 0.3 | 4.3 | 3.83 | 3.67 | 3.58 | 3.53 |
| 0.4 | 3.5 | 3 | 2.83 | 2.75 | 2.7 |
| 0.5 | 3 | 2.5 | 2.33 | 2.25 | 2.2 |
| 0.6 | 2.66 | 2.16 | 2 | 1.91 | 1.87 |
| 0.7 | 2.4 | 1.92 | 1.76 | 1.68 | 1.63 |
| 0.8 | 2.25 | 1.75 | 1.58 | 1.5 | 1.45 |
| 0.9 | 2.11 | 1.61 | 1.44 | 1.36 | 1.31 |
| 1.0 | 2 | 1.5 | 1.33 | 1.25 | 1.2 |
| 1.2 | 1.83 | 1.3 | 1.16 | 1.08 | 1.03 |
| 1.3 | 1.77 | 1.26 | 1.1 | 1.02 | 0.96 |
| 1.6 | 1.62 5 | 1.13 | 0.96 | 0.88 | 0.83 |
| 2.0 | 1.5 | 1 | 0.83 | 0.75 | 0.7 |

Thus, for example, cells positioned deepest within an individual micro-organ are at least about 100 micrometers and not more than about 225-350 micrometers away from a nearest surface of the individual micro-organ, thereby *in* vivo architecture is preserved while at the same time it is ensured that no cell is farther than about 225-300 micrometers from the source of gases and nutrients.

Without being bound by any particular theory, a number of factors provided by the three-dimensional culture system may contribute to its success.

First, the appropriate choice of the explant size, e.g., by use of the above Aleph calculations, provides appropriate surface area to volume ratio for adequate diffusion of nutrients to all cells of the explant, and adequate diffusion of cellular waste away from all cells in the explant.

Second, because of the three-dimensionality of the explant, various cells continue to actively grow, in contrast to cells in monolayer cultures, which grow to confluence, exhibit contact inhibition, and cease to grow and divide. The elaboration of growth and regulatory factors by replicating cells of the explant may be partially responsible for stimulating proliferation and regulating differentiation of cells in culture, e.g., even for the micro-organ which is static in terms of overall volume.

Third, the three-dimensional matrix of the explant retains a spatial distribution of cellular elements which closely approximates that found in the counterpart organ *in vivo.*

Fourth, the cell-cell and cell-matrix interactions may allow the establishment of localized micro-environments conducive to cellular maturation. It has been recognized that maintenance of a differentiated cellular phenotype requires not only growth/differentiation factors but also the appropriate cellular interactions.

While reducing the present invention to practice, and as is further described in the Examples section hereinbelow, it was uncovered that when micro-organs are implanted in a recipient, they provide a sustained dosage of a complex repertoire of angiogenic factors, thus leading to the formation of new blood vessels in the implanted tissues of the host. It was also uncovered that micro-organs can reverse ischemia in host tissues in both normal and aging animals. In addition, it was also uncovered that micro-organs cultured in-vitro also express the same repertoire of angiogenic factors.

Thus, according to one aspect of the present invention there is provided a method of inducing angiogenesis in a tissue of a mammal, such as, for example a human being. The method is effected by implanting at least one micro-organ within the tissue of the mammal. Examples of tissue suitable for micro-organ implantation include but are not limited to, organ tissue or muscle tissue.

Such implantation can be effected via standard surgical techniques or via injecting micro-organ preparations into the intended tissue regions of the mammal utilizing specially adapted syringe employing a needle of a gauge suitable for the administration of micro-organs.

The micro-organs utilized for implantation are preferably prepared from an organ tissue of the implanted mammal or a syngeneic mammal, although xenogeneic tissue can also be utilized for the preparation of the micro-organs providing measures are taken prior to, or during implantation, so as to avoid graft rejection and/or graft versus host disease (GVHD). Numerous methods for preventing or alleviating graft rejection or GVHD are known in the art and as such no further detail is given herein.

According to one preferred embodiment of the present invention, at least a portion of cells of the micro-organ include at least one exogenous polynucleotide sequence. Such polynucleotide sequence(s) are preferably stabily integrated into the genome of these cells although transient polynucleotide sequences can also be utilized. It will be appreciated that such exogenous polynucleotides can be introduced into the cells of the micro-organ following explantation from the organ tissue of the mammal or alternatively the mammal can be transformed with the exogenous polynucleotides prior to preparation of organ tissue explants. Methods for transforming mammalian cells are described in detail hereinbelow.

Such exogenous polynucleotide(s) can serve for enhancing angiogenesis by, for example, up-regulating or down-regulating the expression of one or more endogenous angiogenic factors expressed within these cells. In this case, the polynucleotide(s) can include trans-, or cis-acting enhancer or suppresser elements which regulate either the transcription or translation of the endogenous angiogenic factors expressed within these cells. Numerous examples of suitable translational or transcriptional regulatory elements which can be utilized in mammalian cells are known in the art.

For example, transcriptional regulatory elements are cis or trans acting elements which are necessary for activation of transcription from specific promoters (Carey *et al.* (1989), J. Mol. Biol., 209:423-432; Cress *et al.* (1991) Science, 251:87-90; and Sadowski *et al.* (1988), Nature, 335:563-564).

Translational activators are exemplified by the cauliflower mosaic virus translational activator (TAV). See, for example Futterer and Hohn (1991) EMBO J. 10:3887-3896. In this system a di-cistronic mRNA is produced. That is, two coding regions are transcribed in the same mRNA from the same promoter. In the absence of TAV, only the first cistron is translated by the ribosomes. However, in cells expressing TAV, both cistrons are translated.

The polynucleotide sequence of cis acting regulatory elements can be introduced into cells of micro-organs via commonly practiced gene knock-in techniques. For a review of gene knock-in/out methodology see, for example, United States Patent Nos. 5,487,992, 5,464,764, 5,387,742, 5,360,735, 5,347,075, 5,298,422, 5,288,846, 5,221,778, 5,175,385, 5,175,384, 5,175,383, 4,736,866 as well as Burke and Olson, Methods in Enzymology, 194:251-270, 1991; Capecchi, Science 244:1288-1292, 1989; Davies *et al.,* Nucleic Acids Research, 20 (11) 2693-2698, 1992; Dickinson *et al.*, Human Molecular Genetics, 2(8):1299-1302, 1993; Duff and Lincoln, "Insertion of a pathogenic mutation into a yeast artificial chromosome containing the human APP gene and expression in ES cells", Research Advances in Alzheimer's Disease and Related Disorders, 1995; Huxley *et al.,* Genomics, 9:742-750 1991; Jakobovits *et al.,* Nature, 362:255-261 1993; Lamb *et al.,* Nature Genetics, 5: 22-29, 1993; Pearson and Choi, Proc. Natl. Acad. Sci. USA, 1993, 90:10578-82; Rothstein, Methods in Enzymology, 194:281-301, 1991; Schedl *et al.,* Nature, 362: 258-261, 1993; Strauss *et al.,* Science, 259:1904-1907, 1993, WO 94/23049, WO93/14200, WO 94/06908 and WO 94/28123 also provide information.

Down-regulation of endogenous angiogenic factors can also be achieved via antisense RNA. In this case the exogenous polynucleotide(s) can encode sequences which are complementary to the mRNA sequences of the angiogenic factors transcribed in the cells of the micro-organ. Down regulation can also be effected via gene knock-out techniques.

Up-regulation can also be achieved by overexpressing or by providing a high copy number of one or more angiogenic factor coding sequences. In this case, the exogenous polynucleotide sequences can encode one or more angiogenic factors such as but not limited to VEGF, bFGF, Ang1 or Ang2 which can be placed under a transcriptional control of a suitable promoter of a mammalian expression vector. Suitable mammalian expression vectors include, but are not limited to, pcDNA3, pcDNA3.1(+/-), pZeoSV2(+/-), pSecTag2, pDisplay, pEF/myc/cyto, pCMV/myc/cyto, pCR3.1, which are available from Invitrogen, pCI which is available from Promega, pBK-RSV and pBK-CMV which are available from Stratagene, pTRES which is available from Clontech, and their derivatives.

Numerous methods are known in the art for introducing exogenous polynucleotide sequences into mammalian cells. Such methods include, but are not limited to, direct DNA uptake techniques, and virus or liposome mediated transformation (for further detail see, for example, "Methods in Enzymology" VoL 1-317, Academic Press). Micro-organ bombardment with nucleic acid coated particles is also envisaged.

It will be appreciated that the angiogenic factors expressed in micro-organs can be extracted therefrom as a crude or refined extract in a soluble phase and utilized directly, or as part of a pharmaceutical composition for local administration into host tissues, e.g., via in, in order to induce angiogenesis. It will further be appreciated that since micro-organs express different levels of the various angiogenic factors at different time points following implantation or during culturing, one can extract soluble molecules from different micro-organ cultures at different time points, which when locally administered in a series mimic the temporal expression of an implanted or cultured micro-organ.

Thus, according to another aspect of the present invention, there is provided another method of inducing angiogenesis in a tissue of a mammal. This method is effected by extracting soluble molecules from micro-organs and locally administering at least one predetermined dose of the soluble molecules extracted into the tissue of the mammal Numerous methods of administering are known in the art. Detailed description of some of these methods is given hereinbelow with regards to pharmaceutical compositions.

As mentioned above and according to another preferred embodiment of the present invention the soluble extracts are included in a pharmaceutical composition which also includes a pharmaceutically acceptable carrier which serves for stabilizing and/or enhancing the accessibility or targeting of the soluble extract to target body tissues.

Examples of a pharmaceutically acceptable carrier include but are not limited to, a physiological solution, a viral capsid carrier, a liposome carrier, a micelle carrier, a complex cationic reagent carrier, a polycathion carrier such as poly-lysine and a cellular carrier.

The soluble extract which constitute the "active ingredient" of the pharmaceutical composition can be administered to the individual via various administration modes.

Suitable routes of administration may, for example, include transmucosal or parenteral delivery, including intramuscular, subcutaneous and intramedullary injections as well as intrathecal, direct intraventricular, intravenous, inrtaperitoneal, intranasal, or intraocular injections.

Preferably, the composition or extract is administered in a local rather than a systemic manner, for example, via injection directly into an ischemic tissue region of the individual.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredient may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

The composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active ingredient in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

In addition, the composition of the present invention may be delivered via localized pumps, or time release reservoirs which can be implanted within ischemic tissues of the individual.

Since angiogenic factors are typically secreted from producing cells, micro-organs can also be cultured in suitable media and the conditioned media which includes the secreted angiogenic factors can be collected at predetermined time points and utilized as described hereinabove with respect to the soluble extract.

Thus, according to yet another aspect of the present invention there is provided a method of inducing angiogenesis in a tissue of a first mammal. The method according to this aspect of the present invention is effected by culturing at least one micro-organ in a growth medium to thereby generate a conditioned medium, collecting the conditioned medium following at least one predetermined time period of culturing and administering at least one predetermined dose of the conditioned medium into the tissue of the first mammal to thereby induce angiogenesis in the tissue.

Preferably, the growth medium a minimal essential medium (described hereinabove) which does not contain undefined proteins or other growth factors which may interfere with the intended function of the conditioned media or which may cause undesired reactions in the administered mammal.

It will be appreciated that the collected conditioned media can be processed using chromatographic techniques, such as affinity columns and the like, so as to yield a substantially pure preparation which includes an array of angiogenic factors suitable for inducing angiogenesis when administered to a mammal.

It will further be appreciated that the conditioned medium and the soluble extract described herein can also be derived from micro-organs which include exogenous polynucleotides as described hereinabove. In such cases, if the exogenous polynucleotides utilized encode angiogenic factors, the sequence of such exogenous polynucleotides is selected suitable for the intended administered mammal. For example, in cases where the soluble extract or conditioned medium is administered to humans recipients, human or humanized exogenous polynucleotides are preferably utilized.

The micro-organs according to the teachings of the present invention can be utilized following preparation, or alternatively they can be cryopreserved and stored at -160°C until use. For example, micro-organs can be cryopreserved by gradual freezing in the presence of 10% DMSO (Dimethyl Sulfoxide) and 20% serum.

This can be effected, for example, by encapsulating the micro-organs within planar sheets, (e.g., a semi-permeable matrix such as alginate) and inserting these encapsulated micro-organs into a sealable sterile synthetic plastic bag of dimensions closely similar to that of the encapsulated micro-organs. The bag would contain one plastic tubing input at one end and one plastic tubing output at the opposite end of the bag. The sealed plastic bag containing the planar sheet with the micro-organs could then be perfused with standard culture medium such as Ham's F12 with 10% DMSO and 20% serum and gradually frozen and stored at -160°C.

An important goal in cardiovascular medicine would be to replace surgical bypasses with therapeutic angiogenesis. Yet, in spite of the considerable efficacy observed when angiogenic factors were used in animal models of coronary or limb ischemia, the clinical results have been disappointing. Recently, it has been suggested that clinical failure may be due to the application of the angiogenic factor or the combination of factors utilized. The angiogenesis method of the present invention overcome such limitations of prior art methods.

The present invention brings forth a novel approach which recognizes that angiogenesis is a complex, highly regulated and sustained process, mediated by several regulatory factors. The results presented by the present invention provide a model which allows to study induction of angiogenesis both in and out of the body and as such allows to establish the pattern of expression of key regulatory factors. The results presented herein show that implanted micro-organs express several key angiogenic factors in a coordinated manner both in and out of the body. Furthermore, as shown by the *in-vivo* experiments, micro-organs function as genuine angiopumps not only by transcribing angiogenic factors, but also by inducing the formation of new blood vessels. Furthermore, the magnitude of the induction is such that the vessels formed are sufficient to irrigate the surrounding area and rescue artificially induced hypoxic tissue regions in mice and rats.

The model for ischemia in rats presented hereinbelow in the examples section appears to mimic chronic ischemia since no irreversible damage has occurred. In untreated animals, the ischemia was apparent only after exertion. Presumably, there is enough collateral circulation to keep the limb viable but not enough to allow normal function when faced with an additional challenge. The implantation of micro-organs appears to have reversed this condition by increasing blood supply to ischemic regions. The results show a significant difference between the micro-organ-treated and the control groups which difference is undoubtedly due to induction of angiogenesis by the micro-organs.

In the mice series of *in-vivo* rescue experiments presented herein the ischemic insult was increased. Mice have inferior collateral circulation to the hindlimbs due to less developed tail arteries as compared to rats. In this group, signs of acute irreversible ischemic damage such as gangrene and autoamputation, were detected in the control group. This finding suggests that the present invention may also be useful for salvage procedures, but this issue has to be further tested.

In an additional series of trials presented below the ischemic challenge was increased even further by inducing ischemia in previously diseased animals. Again, irreversible ischemic damage occurred only in the control animals. The damage to the control animals was so severe that there was no point in attempting a stress test. The number of mice was small but the differences were marked. These results are particularly important since they illustrate that micro-organs are capable of inducing angiogenesis even in tissues affected by certain types of peripheral vascular disease.

Thus, the present invention provides methods and compositions for inducing and maintaining blood vessel formation within host tissues for the purposes of rescuing ischemic tissues or generating natural bypasses around blocked blood vessels.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### MATERIAL AND EXPERIMENTAL METHODS

Approval for animal experiments was obtained from the Animal Care and Use Committee of the Faculty of Science of the Hebrew University.

### micro-organ preparation:

Adult animals (C-57 mice or Sprague Dawley rats) were sacrificed by asphyxiation with CO₂ and the lungs were removed under sterile conditions. The lungs were kept on ice and rinsed once with Ringer solution or DMEM including 4.5 gm/l D-glucose. micro-organs were prepared by chopping the lungs with a Sorvall tissue chopper into pieces approximately 300 µm in width. micro-organs were rinsed twice with DMEM containing 100 units/ml Penicillin, 1 mg/ml Streptomycin and 2 mM L-Glutamine (Biological Industries) and kept on ice until use.

### micro-organ implantation:

Adult C-57 mice were anesthetized using 0.6 mg Sodium Pentobarbitol per gram body weight. The mice were shaved, and an incision about 2 cm long was made in the skin at an area above the stomach. A hemostat was used to create subcutaneous "pockets" on both sides of the incision, and 8-9 micro-organs were implanted in each pocket; implantation was done by simply layering the micro-organs over the muscle layer. The incision was sutured and the animals were kept in a warm, lit room for several hours following which they were transferred to the animal house. Four animals were sacrificed at a time interval of either 4 hours, 24 hours, 72 hours or 7 days following implantation and the implanted micro-organs were dissected from surrounding tissues under a surgical microscope and utilized for RNA extraction. The extracted RNA was reverse transcribed and the resulting cDNA was used as a template for PCR analysis using standard methodology. The oligonucleotide primer sequences utilized in the PCR reaction, the expected product size and references are given in Table 2, below.

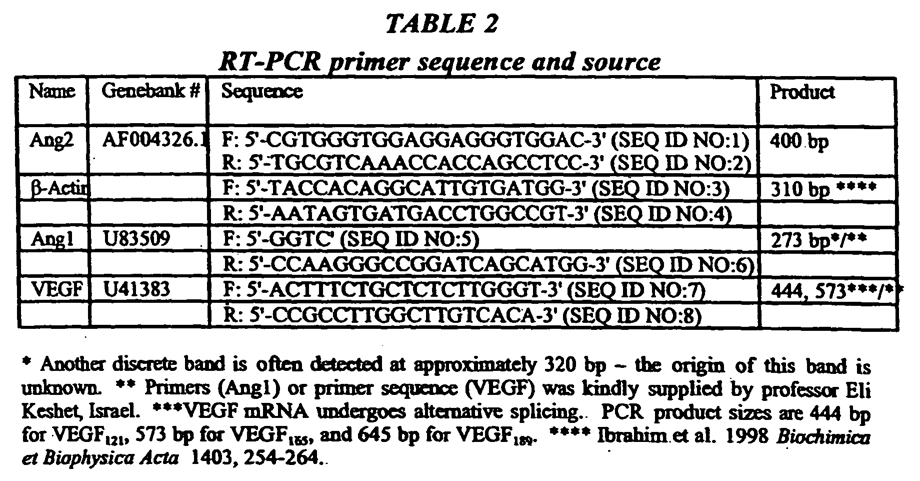

### Densitometric analysis and quantification:

A 10 µl aliquot of each PCR reaction was electrophoresed in a 1.5 % agarose gel stained with ethidium bromide. Gels were imaged utilizing a Macintosh Centris 660 AV computer and a Fujifilm Thermal Imaging System with a Toyo Optics TV zoom lens (75-125 mm, F=1.8, with a Colkin orange 02 filter). Densitometric analysis was performed using the public domain NIH 1.61 analysis software. Quantitation was done by normalizing the expression level of each PCR product to those obtained for β-Actin. All PCR reactions were performed in duplicate. Statistical analysis of the relative expression levels of the various VEGF isoforms was performed by comparison of the means, using Welsh's *t*-test on nonpaired samples before and after implantation.

### Ischemic tissue rescue experiments:

32 Sprague Dawley rats aged 1-4 months and weighing 200-300 grams were utilized. The left common Iliac of each rat was ligated and excised from rats anesthetized using 0.9-1.1 mg/gram body weight of Pentothal at the aortic bifurcation just proximal to the Iliac bifurcation. Sixteen rats were implanted with 3-4 micro-organs each 24 hours following the induction of ischemia. The micro-organs were implanted intramuscularly and subcutaneously along the Femoral artery (medially) and along the sciatic nerve (laterally). The remaining sixteen rats underwent sham implantation 24 hours following the induction of ischemia.

Twenty six C57/B mice aged 1-3 months and weighing 19 to 27 grams were also tested. The left Common Iliac artery of anesthetized mice was ligated and excised at the aortic bifurcation just proximal to the Iliac bifurcation. 3-4 micro-organs were implanted in each mouse at 24 hours following the induction of ischemia. Nine mice were implanted intramuscularly and subcutaneously along the Femoral artery (medially) and along the sciatic nerve (laterally) in the proximal left hindlimb. Seventeen control mice were prepared for implantation following ischemia induction but no implantation was performed. Animals that had venous or nervous damage during the operation as well as those that suffered from significant bleeding were excluded from the trial.

Seven old C57/B mice aged 22 months and weighing 24 to 28 grams also underwent ligation and excision of the left Common Iliac artery as described above. Three were immediately implanted with micro-organs derived from normal healthy syngeneic mice. Four had immediate sham implantation None suffered from venous or nervous damage or had significant bleeding during the operation.

### Functional assay:

The animals were tested on the first and second days following implantation to rule out nerve damage. The test consisted of swimming in a lukewarm water bath which was set at a water level such that the animal needed to constantly exert all four limbs in order to stay afloat. The time limits for exercise were gradually increased. During the first week the time limit was 3 minutes or until efforts to remain afloat ceased. During the second week the limit was raised to 5 minutes, while from the third week onwards the time limit was 6 minutes. A scale from 0 to 10 was created to assess the degree of claudication. A score of 0-1 indicated normal or near normal gait. A score of 2-3 meant slight to moderate claudication with normal weight bearing. A score of 4-5 indicated moderate claudication with disturbance in weight bearing. A score of 6-7 indicated severe claudication. A score of 8-9 indicated a non functioning limb, atrophy or contracture and a score of 10 meant gangrene or autoamputation. The scores were assigned by an independent observer not involved in the experiment and having no knowledge of previous animal treatments.

### Angiography:

Angiography was performed on several rats at days 4, 14, 26 and 31 following implantation. The rats were anesthetized as previously described and a P10 catheter was introduced through the right superficial femoral artery and placed in the aorta. A bolus injection of 1 cc Telebrix was injected and the animal was photographed every 0.5 seconds. Animals undergoing angiography were subsequently excluded from the trial groups.

### EXPERIMENTAL RESULTS

### Implanted micro-organs induce angiogenesis:

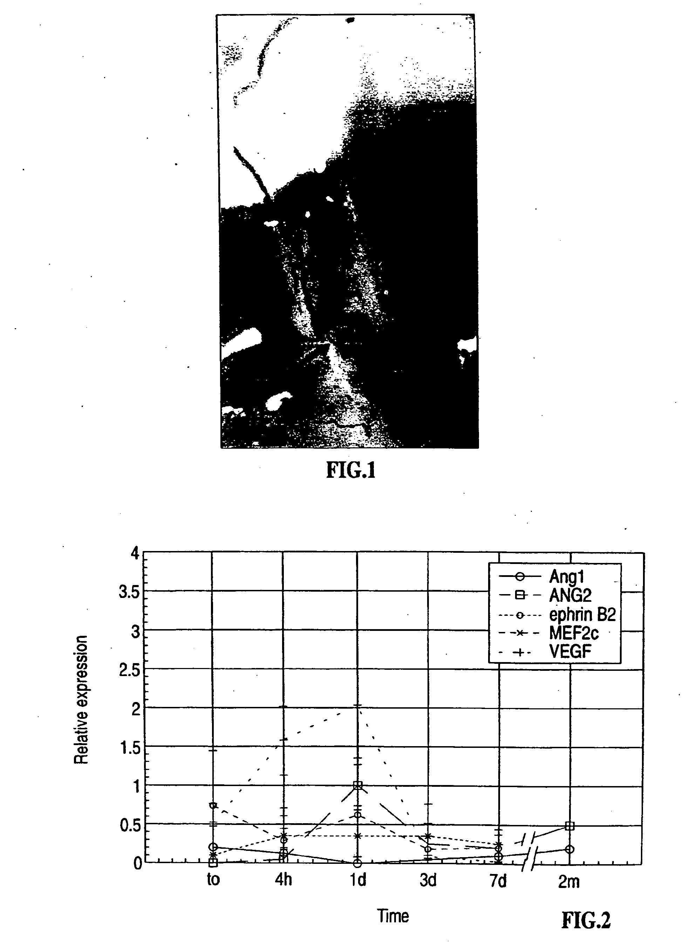

Figure 1 illustrates the response of surrounding tissues to implanted micro-organs. When an micro-organ is implanted subcutaneously into a syngeneic animal, it induces an angiogenic response towards the micro-organ (arrow, Figure 1). A major blood vessel forms and branches into smaller vessels which branch into a net of capillaries which surround the implanted micro-organ.

micro-organs transcribe a sustained and dynamic array of angiogenic growth factors when implanted subcutaneously into syngeneic mice. Figure 2 illustrate a representative semi-quantitative analysis of several known angiogenic growth factors as determined from the RT-PCR analysis performed on RNA extracted from the micro-organs. As seen from the results, a strong induction of angiogenic factor expression occurs at 4 hours post implantation (PI). Following this initial induction, each individual growth factor follows a different expression pattern as is further detailed below.

***VEGF:*** VEGF transcription level continued to rise at 24 hours PI. At three days PI, transcription levels of VEGF decreased. In the days following, lower mRNA levels of this angiogenic factor were detected, which levels were probably necessary in order to maintain the neo-angiogenic state thus formed. At seven days PI, VEGF mRNA returned to a level similar to that detected in micro-organs at the time of implantation (t₀).

***Angiopoietin 1:*** The level of Ang1 mRNA increased for the first 4 hours PI, although variation was high. At one to three days PI, transcription dropped to levels which are even lower than that detected for micro-organs at the time of implantation (t₀) (see Maisonpierre et al., 1997, *Science* 277, 55-60, Gale and Yancopolous, 1999 *Ibid.*). At seven days PI, Ang1 mRNA returned to a level similar to that detected at t₀.

***Angiopoietin 2:*** Ang2, the antagonist of Ang1, was transcribed at high levels at 24 hours PI. mRNA levels dropped at 3 and 7 days PI, although these levels were still higher than the levels detected at t₀, possibly due to ongoing vascular remodeling in and around the implanted micro-organ.

Thus, as is evident from these results, implanted micro-organs transcribe a dynamic array of factors, both stimulators and inhibitors, which participate in the regulation of angiogenesis. This transcription pattern which is responsible for the generation of new blood vessels around the micro-organs is sustained over a period of at least one week PI.

### micro-organs transcribe a sustained and dynamic array of angiogenic growth factors when cultured:

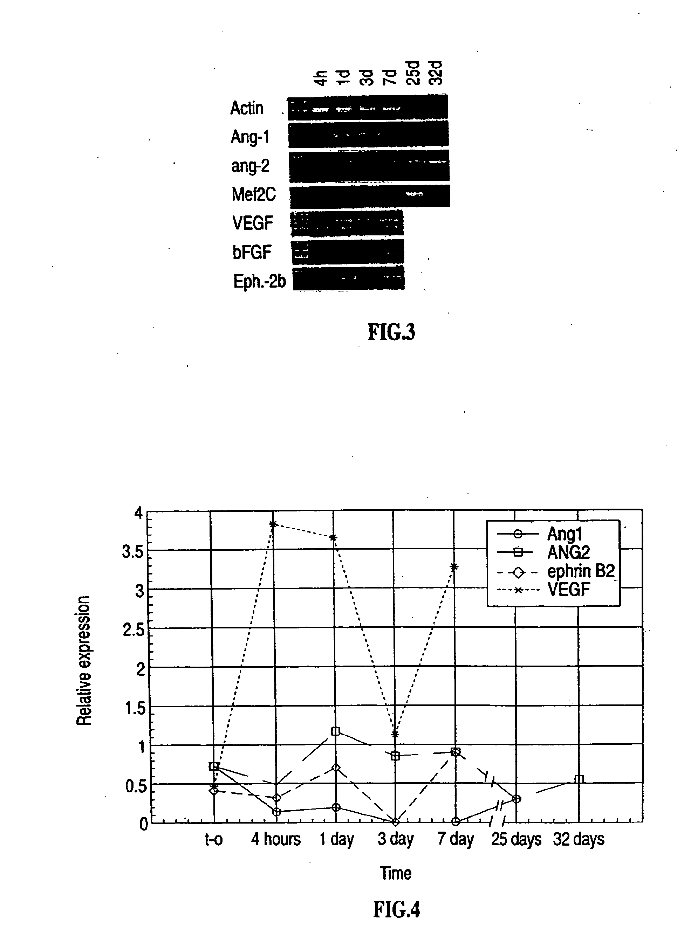

In order to determine the capacity of micro-organs to transcribe angiogenic factors when cultured ex-vivo, micro-organs prepared as described above, were grown in the absence of serum for periods of over one month. Samples were removed at various time points and assayed for the mRNA levels of the several factors. Figure 4 illustrate a representative semi-quantitative analysis of several known angiogenic growth factors as determined from RT-PCR performed on RNA extracted from cultured micro-organs (Figure 3). As shown in both Figures a strong induction of angiogenic factor expression occurs 4 hours following culturing. Following this initial induction, each different growth factor follows a different expression pattern as is described in detail below.

***VEGF:*** VEGF expression levels continued to rise 24 hours after culturing. 3 days after culturing, the expression level of VEGF decreased only to increase again at 7 days PL In the following days expression levels drop and VEGF expression returns to a level comparable to that expressed by micro-organs at the time of culturing.

***Angiopoietin 1:*** The level of Ang1 expression increased for the first 4 hours following culturing although variation was high. Expression dropped 1 to 3 days after culturing to levels even lower than that detected at time of culturing. At seven days after culturing Ang1 expression returned to a level comparable to the level at time of culturing.

***Angiopoietin 2:*** Ang2, the antagonist of Ang1, was expressed at a high level during the first day after culturing. The expression levels were lower 3 and 7 days after culturing, although they are still higher than the expression level at time of culturing.

As is evident from these results, micro-organs which are cultured outside the body remain viable and functional for over a month in vitro and express a dynamic array of angiogenic factors, including both stimulators and inhibitors, which participate in the regulation of angiogenesis.

### Implantation of micro-organs reverse ischemia in limbs of rats and mice:

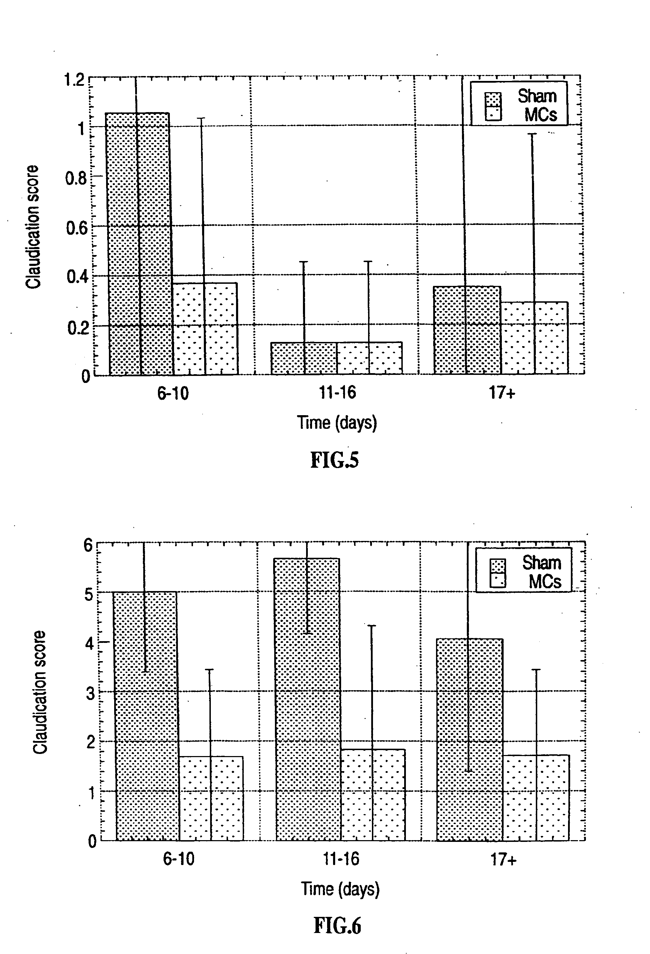

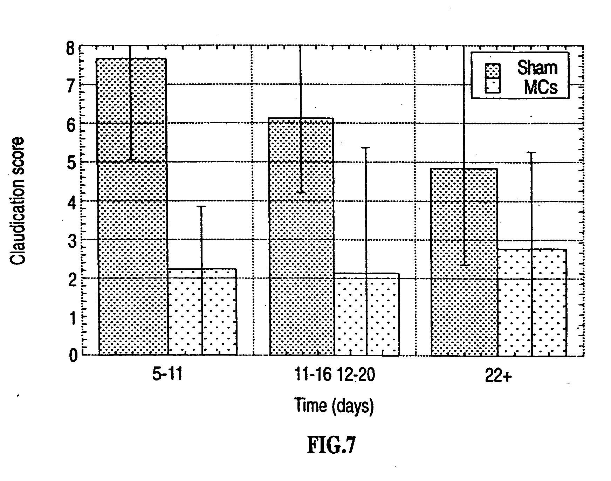

***Series 1:*** The left common iliac artery of thirty two rats was ligated and excised as described above. micro-organs implantation was conducted in sixteen of these rats while the sixteen remaining rats served as the control group (sham operations). All 32 rats survived the operation. No significant difference was detected between the two groups prior to exertion (Figure 5). Following exertion, a significant difference was detected; the cumulated average claudication score for the control group was 4.8 whereas in the micro-organ implanted group the score was 1.6 (Figure 6). Similar results were recorded throughout the study period. The control group scored 5 on days 6-10 post operation (PO), 5 at 11-15 days PO and 4 at day 17 PO. The scores for the micro-organ implanted group were 1.67, 1.5 and 1.7, respectively. It should be noted that the micro-organ implanted group included one rat with an average score of 6.5. A histological examination revealed necrotic micro-organ implants in this rat.
***Series 2:*** Twenty six young C57/B mice were operated as described above without operative damage or preoperative mortality. micro-organ implantation was conducted in nine of these mice while seventeen served as control (sham operations). Of the 17 control mice, 4 developed gangrene on the ischemic-induced limb and died 2-3 days PO (23.5 %). Another mouse from this group had autoamputation of an atrophied limb 8 days after operation (5.9 %). None of the micro-organ implanted mice developed gangrene, autoamputation or postoperative death (0 %). The average cumulated post exertion claudication score for the control group was 6 with scores of 7.7 on days 5-9 PO, 6.2 on days 13-19 PO and 4.1 on days 21-25 PO. The average cumulated claudication score for the micro-organ implanted group was 2.4, with scores of 1.8 on days 5-9 PO, 22 on days 13-19 PO and 3.1 on days 21-25 PO (Figure 7).

### micro-organ implantation rescues ischemic limbs in old mice:

**Stage 3:** Seven aged C57/B mice were operated upon with no operative damage or death. Three mice received micro-organ implants and 4 served as control. Of the control group, 1 developed gangrene and died 3 days PO (25%) and one had autoamputation of an atrophied limb 5 days PO (25%). The remaining two mice had non functioning limbs at rest (a score of 8 on the claudication index). None of the micro-organ implanted mice developed gangrene or autoamputation (0%) and their average claudication score at one week was 5.7 .

### Implanted micro-organs are viable, and vascularized:

In sampled rat specimens the micro-organ implants were viable, with preserved architecture and no evidence of rejection. The micro-organs and surrounding muscle tissue was vascularized via macroscopically visible blood vessels.

### Angiography reveals angiogenic activity in micro-organ-implanted rats:

Angiography was performed on days 4, 14, 26 and 31 PO. There were subtle but detectable differences between the micro-organ-treated groups and the control groups. Evidence of increased angiogenic activity in the implanted limb was detected as early as day 4 PO. New, medium sized blood vessels were visible in the implanted limb sixteen days PO.

### EXAMPLE 2

### Spleen micro-organs

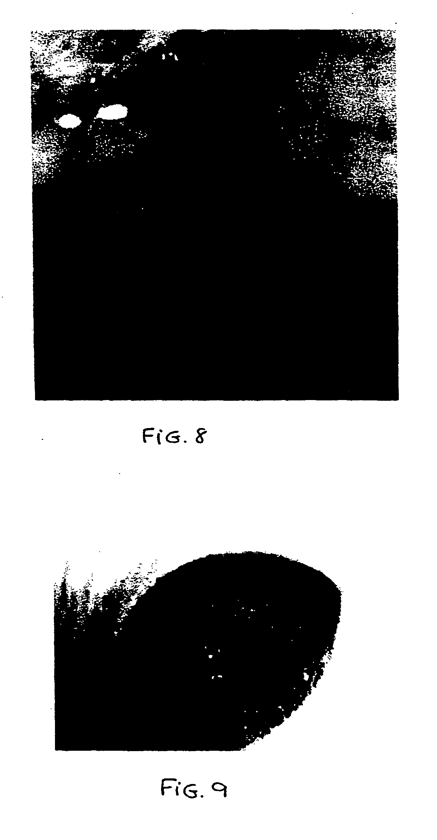

Mouse Spleen micro-organs were prepared from as described hereinabove and implanted into syngeneic mice. Figure 8 illustrates an micro-organ (arrow) which was implanted subcutaneously into the syngeneic mouse and examined at six months following implantation. As is clearly demonstrated in Figure 8, the micro-organ induced angiogenesis. In fact, the pattern of blood vessels formed, gives the impression that the micro-organ is micro-organ was an inherent organ of the host.

### EXAMPLE 3

### Cornea implantation of micro-organs

The cornea is the only tissue of the body which is devoid of blood vessels. As such, the cornea is an excellent model tissue for studying angiogenesis. Rat lung micro-organs were implanted in the corneas of syngeneic rats. As shown in Figure 9, a most remarkable angiogenic pattern was also induced in the cornea. These remarkable results again verify that micro-organs are effective in inducing and promoting angiogenesis.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences disclosed therein and/or identified by a GeneBank accession number mentioned in this specification are herein incorporated in their entirety by reference into the specification, to the same extent as if each individual publication, patent, patent application or sequence was specifically and individually indicated to be incorporated herein by reference. In addition, citation or identification of any reference in this application shall not be construed as an admission that such reference is available as prior art to the present invention.

## Claims

1. A genetically modified micro-organ explant expressing at least one recombinant gene product, the micro-organ explant comprising a population of cells, the micro-organ explant maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the micro-organ explant, at least some of the cells of said population of cells of the micro-organ explant expressing at least one recombinant gene product.

2. The genetically modified micro-organ explant of claim 1, wherein said at least one recombinant gene product is selected from the group consisting of a recombinant protein and a recombinant functional RNA molecule.

3. The genetically modified micro-organ explant of claim 2, wherein said recombinant protein is normally produced by the organ from which the micro-organ explant is derived.

4. The genetically modified micro-organ explant of claim 2, wherein said recombinant protein is normally not produced by the organ from which the micro-organ explant is derived.

5. The genetically modified micro-organ explant of claim 2, wherein said recombinant protein is selected from the group consisting of a protease, a lipase, a ribonuclease, a deoxyribonuclease, a blood clotting factor, a cytochrome p450 enzyme, a transcription factor and a MHC component.

6. The genetically modified micro-organ explant of claim 2, wherein said recombinant protein is selected from the group consisting of a peptide, a glycoprotein and a lipoprotein.

7. The genetically modified micro-organ explant of claim 2, wherein said recombinant protein is selected from the group consisting of insulin, trypsinogen, chymotrypsinogen, carboxypeptidase, triaclyglycerol lipase, phospholipase A₂, elastase, amylase, UDP glucuronyl transferae, ornithine transcarbanoylase, adenosine deaminase, serum thymic factor, thymic humoral factor, thymopoietin, thymosin α₁, gastrin, secretin, cholecystokinin, somatostatin, substance P and growth hormone.

8. The genetically modified micro-organ explant of claim 1, maintainable in culture for at least about twenty-four hours.

9. The genetically modified micro-organ explant of claim 1, having a surface area to volume index **characterized by** the formula 1/x + 1/a > 1.5 mm⁻¹ ; wherein 'x' is a tissue thickness and 'a' is a width of said tissue in millimeters.

10. The genetically modified micro-organ explant of claim 1, wherein said organ is selected from the group consisting of a lymph organ, a pancreas, a liver, a gallbladder, a kidney, a digestive tract organ, a respiratory tract organ, a reproductive organ, skin, a urinary tract organ, a blood-associated organ, a thymus, a spleen.

11. The genetically modified micro-organ explant of claim 1, comprising epithelial and connective tissue cells, arranged in a microarchitecture similar to the microarchitecture of the organ from which the explant was obtained.

12. The genetically modified micro-organ explant of claim 1, wherein the organ is a pancreas, and the population of cells includes islets of Langerhans.

13. The genetically modified micro-organ explant of claim 1, wherein the organ is skin, and the explant includes at least one hair follicle and at least one gland.

14. The genetically modified micro-organ explant of claim 1, wherein the organ is a diseased skin, and the explant includes a population of hyperproliferative or neoproliferative cells from the diseased skin.

15. The genetically modified micro-organ explant of claim 1, wherein the explant is maintainable in a minimal medium.

16. The genetically modified micro-organ explant of claim 1, wherein the explant is maintainable in an artificial medium.

17. The genetically modified micro-organ explant of claim 1, wherein the explant is maintainable in a defined medium.

18. The genetically modified micro-organ explant of claim 1, wherein the retained microarchitecture of the explant comprises one or more cell-cell and cell-matrix orientations between two or more tissues of the organ from which the explant is isolated.

19. The genetically modified micro-organ explant of claim 1, wherein at least a portion of the population of cells is transduced, transformed or transfected with a recombinant construct carrying a recombinant gene encoding said recombinant gene product.

20. The genetically modified micro-organ explant of claim 19, wherein said recombinant construct is a recombinant virus selected from the group consisting of a recombinant hepatitis virus, a recombinant adeno virus, a recombinant adeno-associated virus, a recombinant papilloma virus, a recombinant retrovirus, a recombinant cytomegalovirus and a recombinant simian virus.

21. The genetically modified micro-organ explant of claim 19, wherein said recombinant construct is a naked nucleic acid molecule.

22. The genetically modified micro-organ explant of claim 1, wherein at least a portion of the population of cells are transformed with a foreign nucleic acid sequence via a transformation method selected from the group consisting of calcium-phosphate mediated transfection, DEAE-dextran mediated transfection, electroporation, liposome-mediated transfection, direct injection, and receptor-mediated uptake.

23. A conditioned medium conditioned by the genetically modified micro-organ explant of claim 1 and containing said recombinant gene product.

24. A pharmaceutical preparation comprising the genetically modified micro-organ explant of claim 1.

25. Use of a genetically modified micro-organ for the manufacture of a medicament for producing a micro-organ explant expressing at least one recombinant gene product, comprising the steps of:
(a) isolating from an animal a portion of an organ including a population of cells, the portion of the organ maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the portion of the organ; and
(b) genetically modifying at least some of the cells of said population of cells of the portion of the organ with a recombinant gene to express at least one recombinant gene product.

26. The use of claim 25, wherein said at least one recombinant gene product is selected from the group consisting of a recombinant protein and a recombinant functional RNA molecule.

27. The use of claim 26, wherein said recombinant protein is normally produced by the organ from the micro-organ explant is derived.

28. The use of claim 26, wherein said recombinant protein is normally not produced by the organ from which the micro-organ explant is derived.

29. The use of claim 26, wherein said recombinant protein is selected from the group consisting of a protease, a lipase, a ribonuclease, a deoxyribonuclease, a blood clotting factor, a cytochrome p450 enzyme, a transcription factor and a MHC component.

30. The use of claim 26, wherein said recombinant protein is selected from the group consisting of a peptide, a glycoprotein and a lipoprotein.

31. The use of claim 26, wherein said recombinant protein is selected from the group consisting of insulin, trypsinogen, chymotrypsinogen, carboxypeptidase, triaclyglycerol lipase, phospholipase A₂, elastase, amylase, UDP glucuronyl transferae, ornithine transcarbanoylase, adenosine deaminase, serum thymic factor, thymic humoral factor, thymopoietin, thymosin α₁, gastrin, secretin, cholecystokinin, somatostatin, substance P and growth hormone.

32. The use of claim 25, wherein said genetically modified micro-organ transplant is maintainable in culture for at least about twenty-four hours.

33. The use of claim 25, wherein said genetically modified micro-organ transplant has a surface area to volume index **characterized by** the formula 1/x + 1/a > 1.5 mm⁻¹; wherein 'x' is a tissue thickness and 'a' is a width of said tissue in millimeters.

34. The use of claim 25, wherein said organ is selected from the group consisting of a lymph organ, a pancreas, a liver, a gallbladder, a kidney, a digestive tract organ, a respiratory tract organ, a reproductive organ, skin, a urinary tract organ, a blood-associated organ, a thymus, a spleen.

35. The use of claim 25, wherein said genetically modified micro-organ transplant comprising epithelial and connective tissue cells, arranged in a microarchitecture similar to the microarchitecture of the organ from which the . explant was obtained.

36. The use of claim 25, wherein the organ is a pancreas, and the population of cells includes islets of Langerhans.

37. The of claim 25, wherein the organ is skin, and the explant includes at least one hair follicle and at least one gland.

38. The use of claim 25, wherein the organ is a diseased skin, and the explant includes a population of hyperproliferative or neoproliferative cells from the diseased skin.

39. The use of claim 25, wherein said genetically modified micro-organ transplant is maintainable in a minimal medium.

40. The use of claim 25, wherein the explant is maintainable in an artificial medium.

41. The use of claim 25, wherein the explant is maintainable in a defined medium.

42. The use of claim 25, wherein the retained microarchitecture of the genetically modified micro-organ transplant comprises one or more cell-cell and cell-matrix orientations between two or more tissues of the organ from which the explant is isolated.

43. The use of claim 25, wherein at least a portion of the population of cells is transduced, transformed or transfected with a recombinant construct carrying a recombinant gene encoding said recombinant gene product.

44. The use of claim 43, wherein said recombinant construct is a recombinant virus selected from the group consisting of a recombinant hepatitis virus, a recombinant adeno virus, a recombinant adeno-associated virus, a recombinant papilloma virus, a recombinant retrovirus, a recombinant cytomegalovirus and a recombinant simian virus.

45. The use of claim 43, wherein said recombinant construct is a naked nucleic acid molecule.

46. The use of claim 25, wherein at least a portion of the population of cells are transformed with a foreign nucleic acid sequence via a transformation method selected from the group consisting of calcium-phosphate mediated transfection, DEAE-dextran mediated transfection, electroporation, liposome-mediated transfection, direct injection, and receptor-mediated uptake.

47. Use of a genetically modified micro-organ for the manufacture of a medicament for producing a micro-organ explant expressing at least one recombinant gene product, comprising the step of isolating from a transgenic animal a portion of an organ including a population of cells, the portion of the organ maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the portion of the organ, at least some of the cells of said population of cells of the portion of the organ expressing at least one recombinant gene product.

48. A medical device comprising a polymeric device encapsulating a genetically modified micro-organ explant expressing at least one recombinant gene product, the micro-organ explant comprising a population of cells, the micro-organ explant maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the micro-organ explant, at least some of the cells of said population of cells of the micro-organ explant expressing at least one recombinant gene product.

49. Use of a genetically modified micro-organ for the manufacture of a medicament for delivering a gene product to a recipient, comprising the steps of:
(a) providing a micro-organ explant expressing at least one recombinant gene product, the micro-organ explant comprising a population of cells, the micro-organ explant maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the micro-organ explant, at least some of the cells of said population of cells of the micro-organ explant expressing at least one recombinant gene product; and
(b) implanting the micro-organ explant in the recipient.

50. The use of claim 49, wherein said micro-organ explant is derived from the recipient.

51. The use of claim 49, wherein said micro-organ explant is derived from a donor subject.

52. The use of claim 49, wherein said micro-organ explant is derived from a human being.

53. The use of claim 49, wherein said micro-organ explant is derived from a non-human animal.

54. The use of claim 49, wherein the recipient is a human being.

55. The use of claim 49, wherein the recipient is a non-human animal.

56. The use of claim 49, wherein said at least one recombinant gene product is selected from the group consisting of a recombinant protein and a recombinant functional RNA molecule.

57. The use of claim 56, wherein said recombinant protein is normally produced by the organ from the micro-organ explant is derived.

58. The use of claim 56, wherein said recombinant protein is normally not produced by the organ from which the micro-organ explant is derived.

59. The use of claim 56, wherein said recombinant protein is selected from the group consisting of a protease, a lipase, a ribonuclease, a deoxyribonuclease, a blood clotting factor, a cytochrome p450 enzyme, a transcription factor and a MHC component.

60. The use of claim 56, wherein said recombinant protein is selected from the group consisting of a peptide, a glycoprotein and a lipoprotein.

61. The use of claim 56, wherein said recombinant protein is selected from the group consisting of insulin, trypsinogen, chymotrypsinogen, carboxypeptidase, triaclyglycerol lipase, phospholipase A₂, elastase, amylase, UDP glucuronyl transferae, ornithine transcarbanoylase, adenosine deaminase, serum thymic factor, thymic humoral factor, thymopoietin, thymosin α₁, gastrin, secretin, cholecystokinin, somatostatin, substance P and growth hormone.

62. The use of claim 49, wherein said genetically modified micro-organ transplant is maintainable in culture for at least about twenty-four hours.

63. The use of claim 49, wherein said genetically modified micro-organ transplant has a surface area to volume index **characterized by** the formula 1/x + 1/a > 1.5 mm⁻¹; wherein 'x' is a tissue thickness and 'a' is a width of said tissue in millimeters.

64. The use of claim 49, wherein said organ is selected from the group consisting of a lymph organ, a pancreas, a liver, a gallbladder, a kidney, a digestive tract organ, a respiratory tract organ, a reproductive organ, skin, a urinary tract organ, a blood-associated organ, a thymus, a spleen.

65. The use of claim 49, wherein said genetically modified micro-organ transplant comprising epithelial and connective tissue cells, arranged in a microarchitecture similar to the microarchitecture of the organ from which the explant was obtained.

66. The use of claim 49, wherein the organ is a pancreas, and the population of cells includes islets of Langerhans.

67. The use of claim 49, wherein the organ is skin, and the explant includes at least one hair follicle and at least one gland.

68. The use of claim 49, wherein the organ is a diseased skin, and the explant includes a population of hyperproliferative or neoproliferative cells from the diseased skin.

69. The use of claim 49, wherein said genetically modified micro-organ transplant is maintainable in a minimal medium.

70. The use of claim 49, wherein the explant is maintainable in an artificial medium.

71. The use of claim 49, wherein the explant is maintainable in a defined medium.

72. The use of claim 49, wherein the retained microarchitecture of the genetically modified micro-organ transplant comprises one or more cell-cell and cell-matrix orientations between two or more tissues of the organ from which the explant is isolated.

73. The use of claim 49, wherein at least a portion of the population of cells is transduced, transformed or transfected with a recombinant construct carrying a recombinant gene encoding said recombinant gene product.

74. The use of claim 73, wherein said recombinant construct is a virus vector selected from the group consisting of a recombinant hepatitis virus, a recombinant adeno virus, a recombinant adeno-associated virus, a recombinant papilloma virus, a recombinant retrovirus, a recombinant cytomegalovirus and a recombinant simian virus.

75. The use of claim 73, wherein said recombinant construct is a naked nucleic acid molecule.

76. The use of claim 49, wherein at least a portion of the population of cells are transformed with a foreign nucleic acid sequence via a transformation method selected from the group consisting of calcium-phosphate mediated transfection, DEAE-dextran mediated transfection, electroporation, liposome-mediated transfection, direct injection, and receptor-mediated uptake.

77. The use of claim 49, further comprising the step of encapsulating said genetically modified micro-organ culture prior to said step (c).

78. The use of claim 49, wherein step (a) is effected by:
(i) isolating from an animal a portion of an organ including the population of cells, the portion of the organ maintaining a microarchitecture and a three dimensional structure of an organ from which it is obtained and at the same time having dimensions selected so as to allow diffusion of adequate nutrients and gases to cells in the micro-organ explant and diffusion of cellular waste out of the micro-organ explant so as to minimize cellular toxicity and concomitant death due to insufficient nutrition and accumulation of the waste in the micro-organ explant; and
(ii) genetically modifying at least some of the cells of said population of cells of the portion of the organ with a recombinant gene to express at least one recombinant gene product.

79. The use of claim 49, wherein said step (a) is effected by obtaining said micro-organ explant from an organ of a transgenic animal expressing said recombinant gene product.
